# EUROPEAN PATENT SPECIFICATION

(11) **EP 2 802 598 B1**
(45) Date of publication and mention of the grant of the patent: **06.12.2017**
(21) Application number: 13700347.1
(22) Date of filing: 14.01.2013
(51) Int. Cl.: C07K 14/47, A61K 38/17, A61P 3/10, A61P 3/04

(54) **PEPTIDES AND PEPTIDE DERIVATIVES BASED ON XENIN**
PEPTIDE UND PEPTIDDERIVATE AUF XENINBASIS
PEPTIDES ET DÉRIVÉS PEPTIDIQUES À LA BASE DE XÉNINE

(30) Priority: 12.01.2012 GB 201200509; 04.05.2012 GB 201207895
(43) Date of publication of application: 19.11.2014
(73) Proprietor: University Of Ulster, County Londonderry BT52 1SA (GB)
(72) Inventor: GAULT, Victor Alan, Ballymoney Antrim BT53 6UE Northern Ireland (GB); IRWIN, Nigel, Portrush Antrim BT56 8QJ Northern Ireland (GB); FLATT, Peter, Portstewart Co Londonderry Northern Ireland BT55 7HH (GB)
(74) Representative: Gardner, Rebecca Katherine
(86) International application number: PCT/GB2013/050069
(87) International publication number: WO 2013/104929

(56) References cited:
- WO-A2-2009/108621
- CHRISTINE M.A. MARTIN ET AL: "Degradation, insulin secretion, glucose-lowering and GIP additive actions of a palmitate-derivatised analogue of xenin-25", BIOCHEMICAL PHARMACOLOGY, vol. 84, no. 3, 1 August 2012 (2012-08-01) , pages 312-319, XP55056479, ISSN: 0006-2952, DOI: 10.1016/j.bcp.2012.04.015
- B. M. WICE ET AL: "Xenin-25 Potentiates Glucose-dependent Insulinotropic Polypeptide Action via a Novel Cholinergic Relay Mechanism", JOURNAL OF BIOLOGICAL CHEMISTRY, vol. 285, no. 26, 25 June 2010 (2010-06-25), pages 19842-19853, XP055056411, ISSN: 0021-9258, DOI: 10.1074/jbc.M110.129304
- NIGEL IRWIN ET AL: "GIP(Lys 16 PAL) and GIP(Lys 37 PAL): Novel Long-Acting Acylated Analogues of Glucose-Dependent Insulinotropic Polypeptide with Improved Antidiabetic Potential", JOURNAL OF MEDICINAL CHEMISTRY, vol. 49, no. 3, 1 February 2006 (2006-02-01), pages 1047-1054, XP055044385, ISSN: 0022-2623, DOI: 10.1021/jm0509997

## Description

The present invention relates to peptides and peptide derivatives based on the naturally occurring peptide xenin. Xenin has a role in enhancing insulin secretion and improving glucose homeostasis and the molecules of the invention have utility in the treatment of diabetes and other metabolic conditions including Metabolic Syndrome and obesity.

Type 2 diabetes mellitus (T2DM) is a progressive disease associated with significant morbidity and mortality. In 2010, the International Diabetes Federation (IDF) estimated the global prevalence of T2DM to be 285 million with a forecast to reach 439 million by 2030. With current therapies, glycaemic control remains suboptimal and patients often experience weight gain and hypoglycaemia. There remains a need for new therapeutic agents to tackle T2DM. In general it is necessary to address insulin resistance and insulin deficiency in the patient. Advances in diabetes research have revealed the importance of incretin hormones in maintaining glucose control and patients with T2DM will usually exhibit impaired incretin activity, including impaired secretion of glucagon-like peptide-1 (GLP-1), accelerated metabolism of GLP-1 and glucose-dependent insulinotropic polypeptide (GIP) and defective responsiveness to both hormones.

More recently a further molecule, xenin, a 25 amino acid peptide having the following sequence has been suggested to have a role in enhancing insulin secretion and improving glucose homeostasis, either on its own or through enhancing K-cell mediated regulators of insulin secretion [Taylor et al. J. Endocrinol. 207, 87-93; 2010]. Xenin is co-secreted with GIP from intestinal K-cells. As with other anorectic gastrointestinal peptides, circulating levels of xenin increase after a meal suggesting a possible role in satiety. However, xenin is thought to be degraded in the circulation, making use of the native form of the peptide impractical in terms of a therapeutic agent. A great many strategies exist to increase *in vivo* stability of potential peptidic drugs but the present inventors have prepared specific xenin analogues which exhibit a beneficial array of properties including resistance to degradation in plasma, an enhanced ability to stimulate insulin secretion *in vitro* as compared to xenin itself and a demonstrated ability to lower-glucose levels *in vivo.*

Thus, in a first aspect, the present invention provides a peptide
(i) of SEQ ID No. 1, or
(ii) which is an analogue of SEQ ID No.1 which is able to stimulate insulin secretion and which has no more than 7 amino acid substitutions or deletions as compared to the native sequence (SEQ ID No.1),
the peptide having one or more lysine residues substituted with a lipophilic substituent of 8-40 carbon atoms, optionally via a spacer.

Thus, the invention provides a peptide of SEQ ID No. 1 or an analogue thereof which is able to stimulate insulin secretion and which has no more than 7 amino acid substitutions or deletions as compared to the native sequence, the peptide having one or more lysine residues substituted with a lipophilic substituent of 8-40 carbon atoms, optionally via a spacer.

In preferred embodiments, the peptide has no more than 6, 5, 4, 3, 2, 1 or most preferably no substitutions or deletions.

Methods for assessing the ability of a molecule to stimulate insulin secretion are known in the art and the effect may be *in vivo* and/or *in vitro.* The Examples provide a convenient method to assess insulin secretion utilising BRIN-BD11 cells. The analogues preferably exhibit at least 30%, more preferably at least 50% or at least 70%, most preferably at least 90% or at least 100%, of the ability of native xenin to stimulate insulin secretion. In preferred embodiments the analogues exhibit greater than 100% of the activity of native xenin, for example as assessed in an *in vitro* assay using BRIN-BD11 cells (ECACC accession number 100330033).

The peptide may have a lipophilic moiety attached to more than one of the lysine residues contained therein but preferably only one lysine residue will be so modified, preferably Lys₁₃.

The lysine residue(s) is substituted with a lipophilic substituent at the ε-amino group. A carboxyl group of the lipophilic substituent may form an amide bond with the ε-amino group. The lipophilic substituent preferably comprises 8-24 carbon atoms. Preferably the lipophilic substituent is derived from a fatty acid which may be saturated or unsaturated, preferably saturated. Suitably fatty acids include: lauric, myristic, palmitic, stearic, behenic, palmitoleic, oleic, linoleic, α-linoleic, γ-linoleic and arachidonic acid.

In a preferred embodiment the lipophilic substituent may be attached to the lysine residue by a spacer, for example in such a way that a carboxyl group of the spacer forms an amide bond with the ε-amino group of lysine. In a preferred embodiment, the spacer is an α,ω-amino acid. Examples of suitable spacers are succinic acid, Lys, Glu or Asp, or a dipeptide such as Gly-Lys. When the spacer is succinic acid, one carboxyl group thereof may form an amide bond with the amino group of lysine, and the other carboxyl group thereof may form an amide bond with an amino group of the lipophilic substituent. When the spacer is Lys, Glu or Asp, the carboxyl group thereof may form an amide bond with the amino group of lysine, and the amino group thereof may form an amide bond with a carboxyl group of the lipophilic substituent. When Lys is used as the spacer, a further spacer may in some instances be inserted between the ε-amino group of Lys and the lipophilic substituent. In one preferred embodiment, such a further spacer is succinic acid which forms an amide bond with the ε-amino group of Lys and with an amino group present in the lipophilic substituent. In another preferred embodiment such a further spacer is Glu or Asp which forms an amide bond with the ε-amino group of Lys and another amide bond with a carboxyl group present in the lipophilic substituent, that is, the lipophilic substituent is a N^{ε}-acylated lysine residue. Other preferred spacers are N^{ε}-(γ-L-glutamyl), N^{ε}-(β-L-asparagyl), N^{ε}-glycyl, and N^{ε}-(α-(γ-aminobutanoyl). Glu is particularly preferred as a spacer.

In another preferred embodiment of the present invention, the lipophilic substituent has a group which can be negatively charged. One preferred such group is a carboxylic acid group. In a further preferred embodiment, the lipophilic substituent is attached to the parent peptide by means of a spacer which is an unbranched alkane, e.g. α, Ω-dicarboxylic acid group having from 1 to 7 methylene groups, preferably two methylene groups which spacer forms a bridge between the ε-amino group of lysine and an amino group of the lipophilic substituent. Further suitable spacers are described in US 6,268,343. Thus the lipophilic substituent may be an acyl group of formula CH₃(CH₂)ₙCO- wherein n is an integer from 6 to 38, preferably an integer from 6 to 22, e.g. CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- or CH₃(CH₂)₂₂CO-.

In a further preferred embodiment, the lipophilic substituent is an acyl group of a straight-chain or branched alkane, α,ω-dicarboxylic acid.

In a further preferred embodiment, the lipophilic substituent is an acyl group of the formula HOOC(CH₂)*ₘ*CO-, wherein m is an integer from 6 to 38, preferably an integer from 6 to 24, more preferably HOOC(CH₂)₁₄CO-, HOOC(CH₂)₁₆CO-, HOOC(CH₂)₁₈CO-, HOOC(CH₂)₂₀CO- or HOOC(CH₂)₂₂CO-.

In a particularly preferred embodiment, the peptide is xenin (SEQ ID No.1) having a (γ-L-Glutamoyl(N-α-hexadecanoyl)) modification on lysine (K) 13. The epsilon nitrogen of the lysine residue is substituted with the (γ-L-Glutamoyl(N-α-hexadecanoyl)) modification.

Without wishing to be bound by theory, the lipophilic substituents may self-aggregate and/or non-covalently bind to plasma albumin fatty acid binding sites. This results in slower absorption and prolongs the half-life of the peptides.

In a further aspect the *in vivo* stability of xenin is increased by modification of the amino acid sequence. Thus, in a further aspect, the present invention provides a peptide
(i) of SEO ID No. 1; or
(ii) which is an analogue of SEQ ID No.1 which is able to stimulate insulin secretion and which has no more than 7 amino acid substitutions or deletions as compared to the native sequence (SEQ ID No.1);
in which one or more of Lys₄, Lys₈, Arg₁₁, Lys₁₃, Phe₁₇, Lys₂₀, or Arg₂₁ have been replaced with another amino acid which increases resistance of the peptide to enzymatic degradation.

Thus, the invention provides a peptide of SEQ ID No. 1 or an analogue thereof which is able to stimulate insulin secretion and which has no more than 7 amino acid substitutions or deletions as compared to the native sequence and in which one or more (i.e. 1, 2, 3, 4, 5, 6 or 7) of:

Lys₄

Lys₈

Arg₁₁

Lys₁₃

Phe₁₇

Lys₂₀

Arg₂₁

have been replaced with another amino acid (genetically encoded or non-genetically encoded) which increases resistance of the peptide to enzymatic degradation. Any number or combination of Lys₄, Lys₈, Arg₁₁, Lys₁₃, Phe₁₇, Lys₂₀ and Arg₂₁ may be replaced.

Lys₄, Lys₈, Arg₁₁, Lys₁₃, Phe₁₇, Lys₂₀, Arg₂₁ are preferably replaced by glutamine or isoleucine. Typically one to three of these amino acids have been replaced.

Suitable methods for determining susceptibility of a peptide to enzymatic degradation are known in the art and described, for example, in Taylor *et al. supra* and in the present Example. *In vitro* or *in vivo* methods can be used to confirm increased resistance to enzymatic cleavage but, for convenience, *in vitro* methods are preferred.

Preferred peptides will have a non-native amino acid at one or more of positions 8, 11, 13 and 17. Said non-native amino acid will reduce the susceptibility of the peptide to cleavage between, respectively, positions 8 and 9, 10 and 11, 13 and 14 and 17 and 18.

Other preferred peptides have non-native amino acids at one or more (i.e. 1, 2, 3, 4, 5 or 6) of positions 4, 8, 11, 13, 20 and 21. Particularly preferred peptides have non-native amino acids at each of positions 4, 8, 11, 13, 20 and 21. Lys₄, Lys₈, Arg₁₁, Lys₁₃, Lys₂₀, Arg₂₁ are preferably replaced by glutamine or isoleucine, more preferably glutamine. The peptide set forth as SEQ ID No. 16 is a particularly preferred peptide. SEQ ID NO:16 is also referred to herein as xenin-Gln.

Preferred peptides of the invention include:
MLTKFETQSARVKGLSFHPKRPWIL Gln8 (SEQ ID No. 2)
MLTKFETISARVKGLSFHPKRPWIL Ile8 (SEQ ID No. 3)
MLTKFETKSAQVKGLSFHPKRPWIL Gln11 (SEQ ID No. 4)
MLTKFETKSAIVKGLSFHPKRPWIL Ile11 (SEQ ID No. 5)
MLTKFETKSARVQGLSFHPKRPWIL Gln13 (SEQ ID No. 6)
MLTKFETKSARVIGLSFHPKRPWIL Ile13 (SEQ ID No. 7)
MLTKFETKSARVKGLSQHPKRPWIL Gln17 (SEQ ID No. 8)
MLTKFETKSARVKGLSIHPKRPWIL Ile17 (SEQ ID No. 9)
MLTQFETKSARVKGLSFHPKRPWIL Gln4 (SEQ ID No. 10)
MLTIFETKSARVKGLSFHPKRPWIL Ile4 (SEQ ID No. 11)
MLTKFETKSARVKGLSFHPQRPWIL Gln20 (SEQ ID No. 12)
MLTKFETKSARVKGLSFHPIRPWIL Ile20 (SEQ ID No. 13)
MLTKFETKSARVKGLSFHPKQPWIL Gln21 (SEQ ID No. 14)
MLTKFETKSARVKGLSFHPKIPWIL Ile21 (SEQ ID No. 15)
MLTQFETQSAQVQGLSFHPQQPWIL Gln4, 8, 11, 13, 20 and 21 (SEQ ID No.16)

The three letter amino acid code and its associated number(s) that appears after the end of the above preferred peptide sequences (e.g. "Gln8" for SEQ ID NO: 2) indicates the replacement amino acid residue and its position.

These two strategies for increasing the *in vivo* half life of xenin and related molecules can be combined and thus preferred analogues of xenin according to the present invention are those in which one or more Lys residues incorporate a lipophilic substituent as defined herein and one or more of Lys₄, Lys₈, Arg₁₁, Lys₁₃, Phe₁₇, Lys₂₀ and Arg₂₁ have been replaced.

In a further aspect the present invention provides molecules comprising peptides of the invention as described above.

In all cases, the amino acids may be in D or L form.

The term 'peptides' as used herein also encompasses standard peptidomimetic equivalents thereof. A peptidomimetic is typically characterised by retaining the polarity, three dimensional size and functionality of its peptide equivalent but wherein the peptide bonds have been replaced, often by more stable linkages. By 'stable' is typically meant more resistant to enzymatic degradation by hydrolytic enzymes. Generally, the bond which replaces the amide bond (amide bond surrogate) conserves many of the properties of the amide bond, e.g. conformation, steric bulk, electrostatic character, possibility for hydrogen bonding etc. Chapter 14 of "Drug Design and Development", Krogsgaard, Larsen, Liljefors and Madsen (Eds) 1996, Horwood Acad. Pub provides a general discussion of techniques for the design and synthesis of peptidomimetics. Suitable amide bond surrogates include the following groups: N-alkylation (Schmidt, R. et al., Int. J. Peptide Protein Res., 1995, 46,47), retro-inverse amide (Chorev, M and Goodman, M., Acc. Chem. Res, 1993, 26, 266), thioamide (Sherman D.B. and Spatola, A.F. J. Am. Chem. Soc., 1990, 112, 433), thioester, phosphonate, ketomethylene (Hoffman, R.V. and Kim, H.O. J. Org. Chem., 1995, 60, 5107), hydroxymethylene, fluorovinyl (Allmendinger, T. et al., Tetrahydron Lett., 1990, 31, 7297), vinyl, methyleneamino (Sasaki, Y and Abe, J. Chem. Pharm. Bull. 1997 45, 13), methylenethio (Spatola, A.F., Methods Neurosci, 1993, 13, 19), alkane (Lavielle, S. et. al., Int. J. Peptide Protein Res., 1993, 42, 270) and sulfonamido (Luisi, G. et al. Tetrahedron Lett. 1993, 34, 2391).

Suitable peptidomimetics include reduced peptides where the amide bond has been reduced to a methylene amine by treatment with a reducing agent e.g. borane or a hydride reagent such as lithium aluminium hydride. Such a reduction has the added advantage of increasing the overall cationicity of the molecule.

Other peptidomimetics include peptoids formed, for example, by the stepwise synthesis of amide-functionalised polyglycines. Some peptidomimetic backbones will be readily available from their peptide precursors, such as peptides which have been permethylated, suitable methods are described by Ostresh, J.M. et al. in Proc. Natl. Acad. Sci. USA 1994, 91, 11138-11142. Strongly basic conditions will favour N-methylation over O-methylation and result in methylation of some or all of the nitrogen atoms in the peptide bonds and the N-terminal nitrogen. Preferred peptidomimetic backbones include polyesters, polyamines and derivatives thereof as well as substituted alkanes and alkenes.

In a further aspect the present invention provides the peptides defined herein for use in therapy, in particular for use in the treatment of T2DM, alternatively or in addition, for the treatment of insulin dependent diabetes mellitus, insulin resistance, Metabolic Syndrome, or obesity. Treatment includes prophylactic treatment.

Also disclosed herein is a method of treating T2DM, insulin dependent diabetes mellitus, insulin resistance, Metabolic Syndrome or obesity which method comprises administering to a patient in need thereof a therapeutically effective amount of a peptide of the invention as defined herein.

A therapeutically effective amount will be determined based on the clinical assessment and can be readily monitored, *in vivo,* in relation to the hormones involved in regulation of glucose levels or glucose itself.

Also disclosed herein is the use of a peptide of the invention as defined herein in the manufacture of a medicament for treating T2DM, insulin dependent diabetes mellitus, insulin resistance, Metabolic Syndrome or obesity.

These treatments may involve co-administration with another antidiabetic agent, e.g. insulin or an incretin or mimic or analogue thereof (e.g. GIP or (D-Ala²)GIP) or another agent for treatment of Metabolic Syndrome or insulin resistance or another antiobesity agent. Other combinations of agents for use in treating the conditions discussed herein are native xenin and GIP, and native xenin and (D-Ala²)GIP).

Subjects treated in accordance with the present invention will preferably be humans but veterinary treatments are also contemplated.

The peptides of the invention may be generated using recombinant DNA technology but will preferably be prepared using chemical synthesis so that the peptide is generated by stepwise elongation, one amino acid at a time.

In building up the peptide, one can in principle start either at the C-terminus or the N-terminus, although a C-terminal starting procedure is preferred.

Methods of peptide synthesis are well known in the art but for the present invention it may be particularly convenient to carry out the synthesis on a solid phase support, such supports being well known in the art. Generally the reactive groups present (for example amino, thiol and/or carboxyl) will be protected during overall synthesis. The final step in the synthesis will thus be the deprotection of a protected derivative of the invention.

A wide choice of protecting groups for amino acids are known and suitable amine protecting groups may include carbobenzoxy (also designated Z) t-butoxycarbonyl (also designated Boc), 4-methoxy-2,3,6-trimethylbenzene sulphonyl (Mtr) and 9-fluorenylmethoxy-carbonyl (also designated Fmoc). It will be appreciated that when the peptide is built up from the C-terminal end, an amine-protecting group will be present on the α-amino group of each new residue added and will need to be removed selectively prior to the next coupling step.

A wide range of procedures exists for removing amine- and carboxyl-protecting groups. These must, however, be consistent with the synthetic strategy employed. The side chain protecting groups must be stable to the conditions used to remove the temporary α-amino protecting group prior to the next coupling step.

Amine protecting groups such as Boc and carboxyl protecting groups such as tBu may be removed simultaneously by acid treatment, for example with trifluoroacetic acid. Thiol protecting groups such as Trt may be removed selectively using an oxidation agent such as iodine.

Formulations comprising one or more peptides of the invention in admixture with a suitable diluent, carrier or excipient constitute a further aspect of the present invention. Such formulations may be for pharmaceutical or veterinary use. Suitable diluents, excipients and carriers are known to the skilled man.

The compositions according to the invention may be presented, for example, in a form suitable for oral, nasal, parenteral, intravenal, topical or rectal administration.

The active compounds defined herein may be presented in the conventional pharmacological forms of administration, such as tablets, coated tablets, nasal sprays, solutions, emulsions, liposomes, powders, capsules or sustained release forms. Conventional pharmaceutical excipients as well as the usual methods of production may be employed for the preparation of these forms.

Injection solutions may, for example, be produced in the conventional manner, such as by the addition of preservation agents, such as p-hydroxybenzoates, or stabilizers, such as EDTA. The solutions are then filled into injection vials or ampoules.

Nasal sprays may be formulated similarly in aqueous solution and packed into spray containers, either with an aerosol propellant or provided with means for manual compression.

The pharmaceutical compositions (formulations) of the present invention are preferably administered parenterally. Parenteral administration may be performed by subcutaneous, intramuscular or intravenous injection by means of a syringe, optionally a pen-like syringe. Alternatively, parenteral administration can be performed by means of an infusion pump. A further option is a composition which may be a powder or a liquid for the administration of the peptide in the form of a nasal or pulmonal spray. As a still further option, the peptide of the invention can also be administered transdermally, e.g. from a patch, optionally a iontophoretic patch, or transmucosally, e.g. bucally.

Dosage units containing the peptides preferably contain 0.1-10mg, for example 1-5mg of the active agent. The pharmaceutical compositions may additionally comprise further active ingredients as described above in the context of co-administration regimens.

The invention will now be further described with reference to the following non-limiting Examples and figures in which:

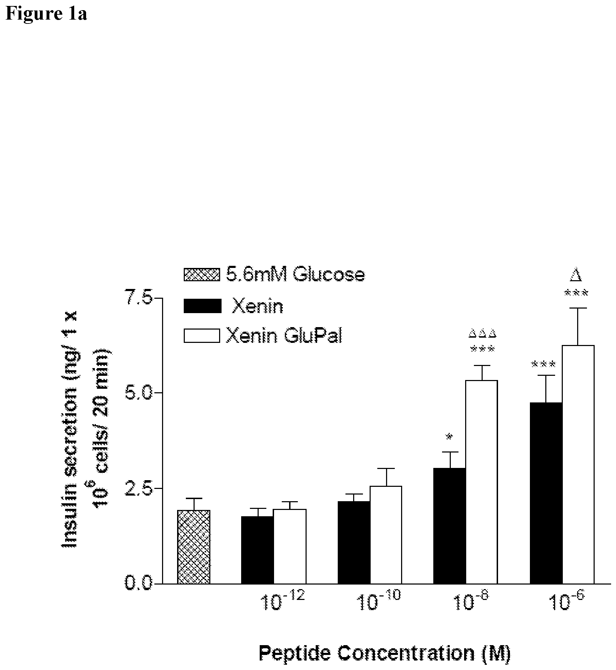

**Figure 1a****:** BRIN-BD11 cells were incubated with a range of peptide concentrations (n = 8; 20 min) in 5.6 mM glucose. Radioimmunoassay was used to measure insulin secretion. Values represent means±SEM. ^{*}P<0.05, ^{***}P<0.001 in comparison with 5.6 mM glucose control. ^{Δ}P<0.05 and ^{ΔΔΔ}P<0.001 compared with native xenin alone.

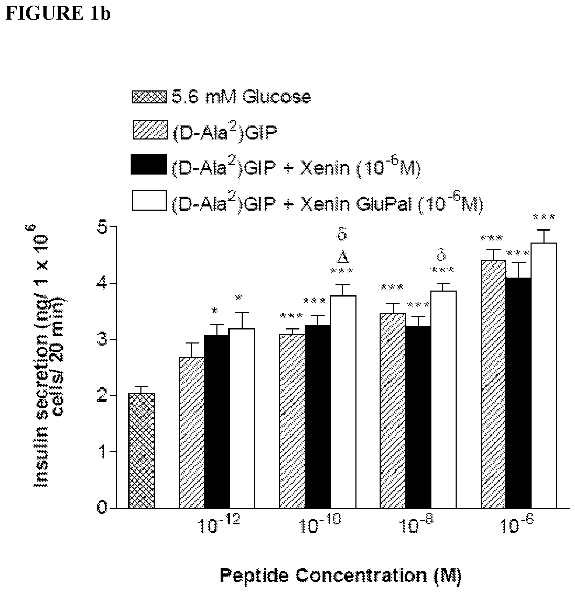

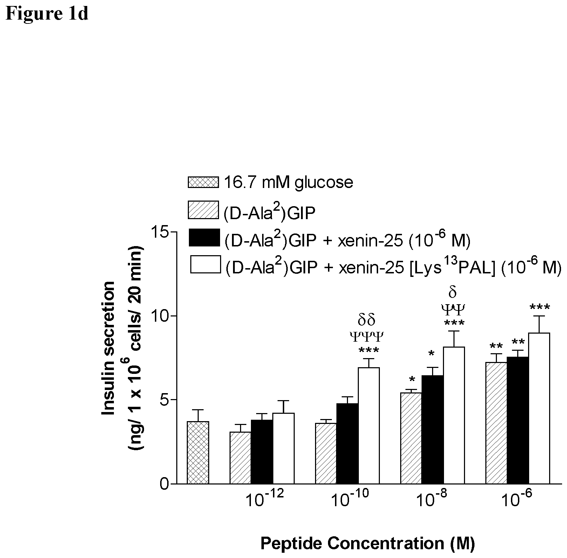

**Figure 1b** **and** **Figure 1d****:** BRIN-BD11 cells were incubated with a range of (D-Ala²)GIP concentrations in the presence of native xenin or xenin-GluPal at 5.6 mM glucose (Figure 1b) or 16.7mM glucose (Figure 1d) (n = 8; 20 min). Radioimmunoassay was used to measure insulin secretion. Values represent means±SEM. ^{*}P<0.05 and ^{***}P<0.001 in comparison to 5.6 mM glucose control. ^{Δ}P<0.05 compared with (D-Ala²)GIP alone. ^{δ}P<0.05 compared to (D-Ala²)GIP + xenin (10⁻⁶ M). ^{**}P<0.01 compared to 5.6mM glucose control. ^{ψψ}P<0.01 and ^{ψψψ}P<0.001 compared to (D-Ala²)GIP. ^{δδ}P<0.01 compared to xenin-25 in combination with (D-Ala²)GIP. Xenin-GluPal and xenin-25[Lys¹³PAL] are synonyms.

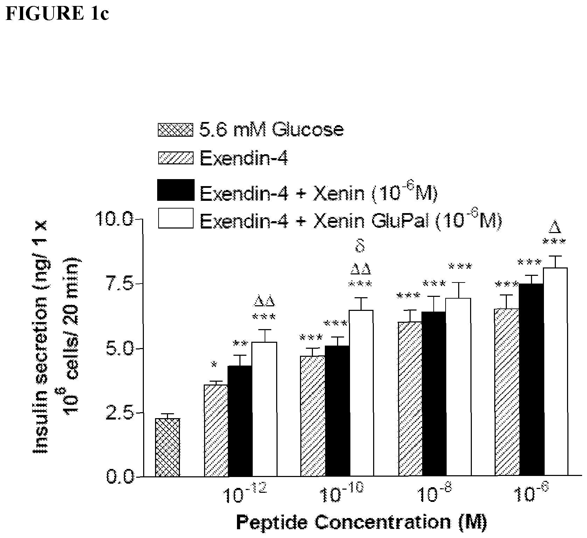

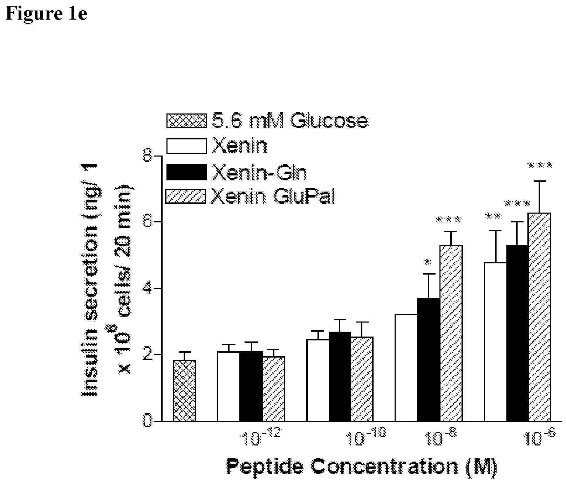

**Figure 1** **c:** BRIN-BD11 cells were incubated with a range of exendin-4 concentrations in the presence of xenin or xenin-GluPal at 5.6 mM glucose (n = 8; 20 min). Radioimmunoassay was used to measure insulin secretion. Values represent means± SEM. ^{*}P<0.05, ^{**}P<0.01, ^{***}P<0.001 in comparison with 5.6 mM glucose control. ^{Δ}P<0.05 and ^{ΔΔ}P<0.01 compared with exendin-4 alone. ^{δ}P<0.05 compared to exendin-4 + xenin (10⁻⁶ M).
**Figure 1e****:** BRIN-BD11 cells were incubated with a range of peptide concentrations (n = 8; 20 min) in 5.6 mM glucose. Radioimmunoassay was used to measure insulin secretion. Values represent means±SEM. *P<0.05, **P<0.01, ***P<0.001 in comparison with 5.6 mM glucose control.

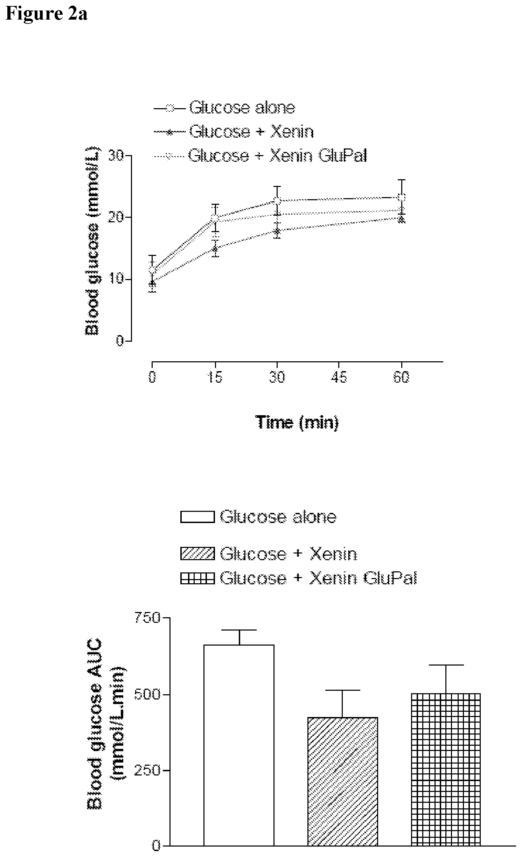

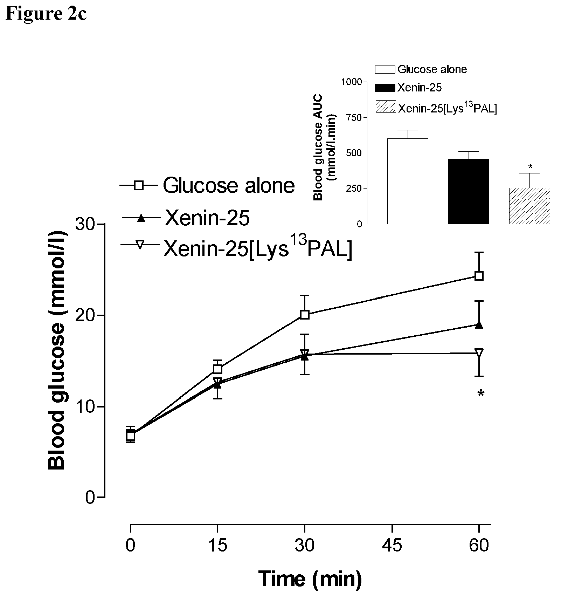

**Figure 2a** **and** **Figure 2c****:** shows the acute glucose-lowering actions of native xenin and xenin-GluPal in high-fat fed mice (Figure 2a) and normal mice (Figure 2c). Plasma glucose was measured prior to (t = 0) and 15, 30, 60 min after i.p. injection of glucose alone (18 mmol/kg) or in combination with native xenin or xenin-GluPal (each at 25 nmol/kgbody weight). Plasma glucose AUC values are also shown. Xenin-GluPal and xenin-25[Lys¹³PAL] are synonyms.

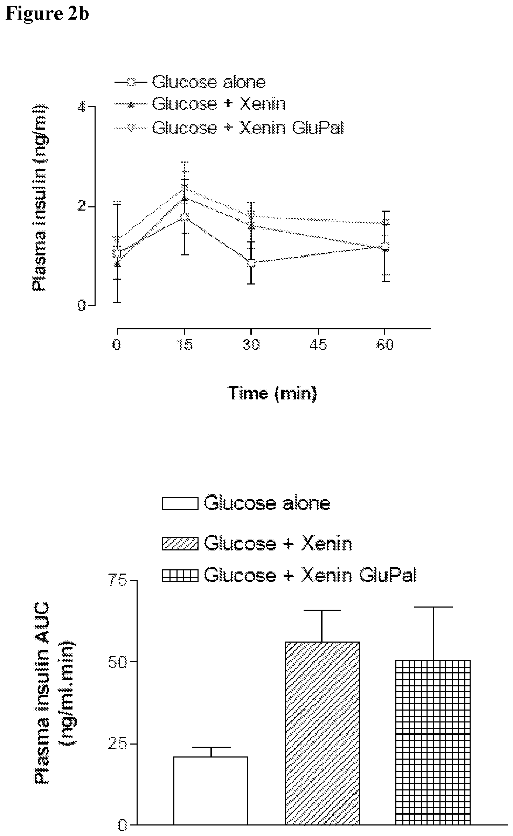

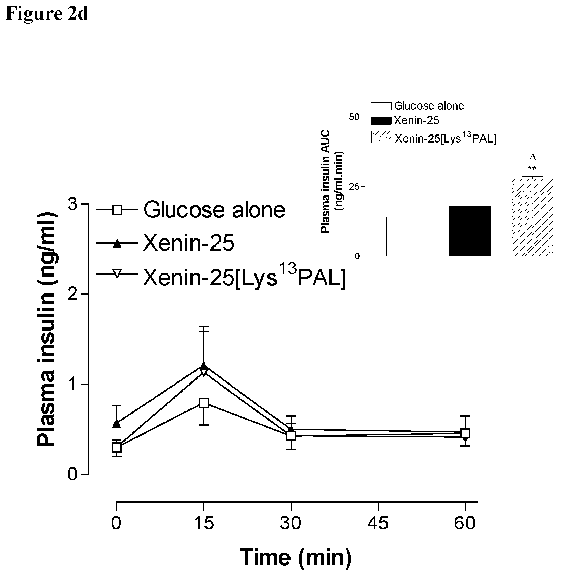

**Figure 2b** **and** **Figure 2d****:** shows the acute insulinotropic actions of native xenin and xenin-GluPal in high-fat fed mice (Figure 2b) and normal mice (Figure 2d). Plasma insulin was measured prior to (t = 0) and 15, 30, 60 min after i.p. injection of glucose alone (18 mmol/kg) or in combination with native xenin or xenin-GluPal (each at 25 nmol/kgbody weight). Plasma insulin AUC values are also shown. Xenin-GluPal and Xenin-25[Lys¹³PAL] are synonyms.

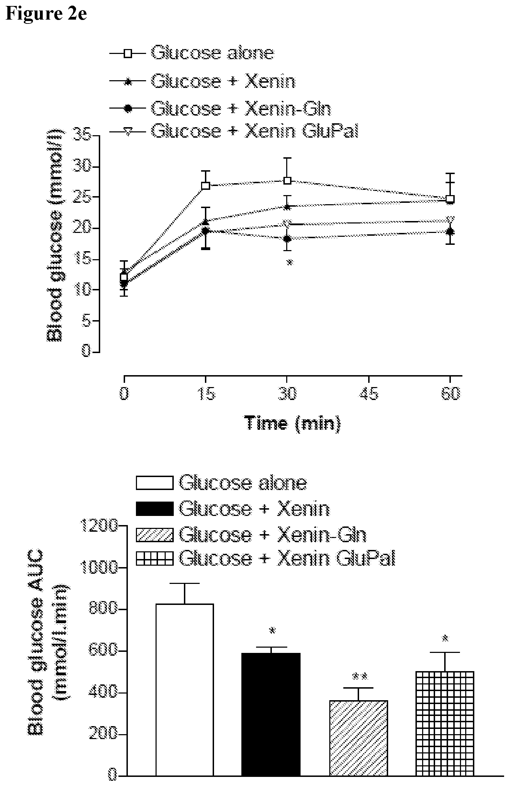

**Figure 2e****:** shows the acute glucose-lowering actions of native xenin, xenin-GluPal and Xenin-Gln in high-fat fed mice. Plasma glucose was measured prior to (t = 0) and 15, 30, 60 min after i.p. injection of glucose alone (18 mmol/kg) or in combination with xenin, xenin-GluPal and Xenin-Gln (each at 25 nmol/kgbody weight). Plasma glucose AUC values are also shown. *P<0.05 and **P<0.01 compared to glucose alone.

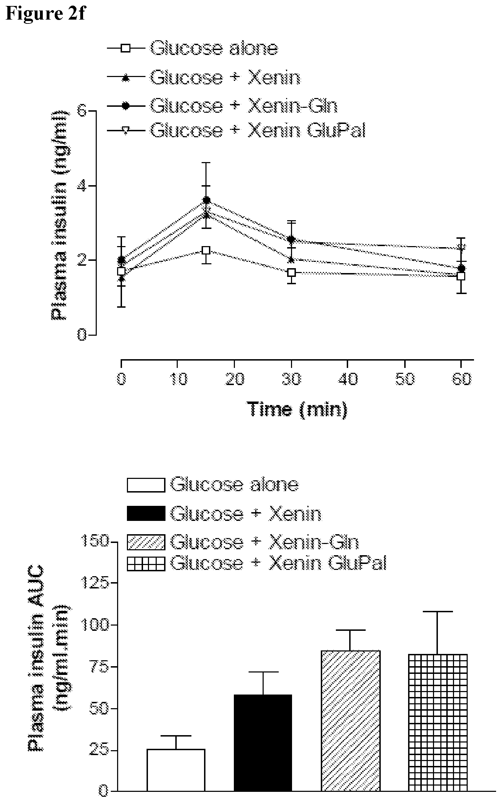

**Figure 2f****:** shows the acute insulinotropic actions of native xenin, xenin-GluPal and Xenin-Gln in high-fat fed mice. Plasma insulin was measured prior to (t = 0) and 15, 30, 60 min after i.p. injection of glucose alone (18 mmol/kg) or in combination with native xenin, xenin-GluPal and Xenin-Gln (each at 25 nmol/kgbody weight). Plasma insulin AUC values are also shown.

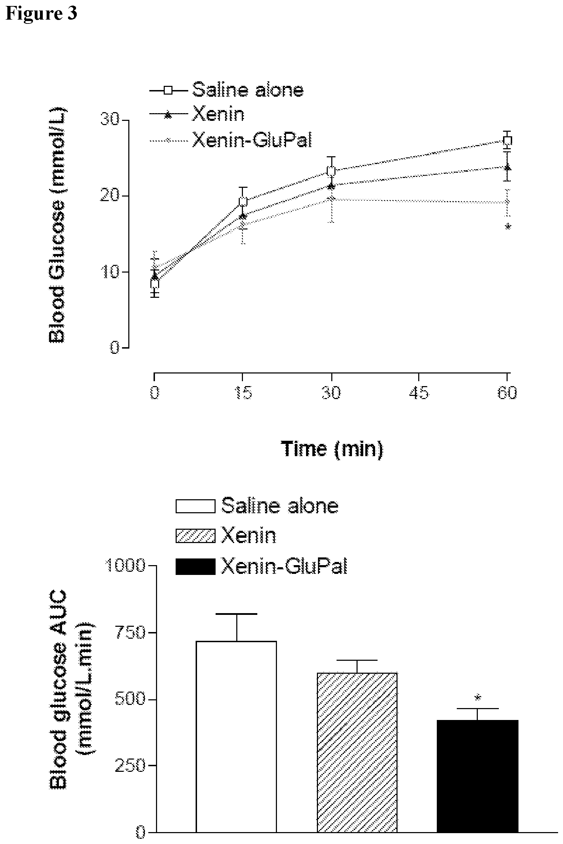

**Figure 3****:** shows the persistent glucose-lowering actions of native xenin and xenin-GluPal in high-fat fed mice. Blood glucose was measured prior to (t=0) and 15, 30 and 60 min after i.p. injection of glucose alone (18mmol/kg body weight) following the administration of saline alone (0.9% w/v NaCl), xenin-25 (i.e. native xenin) or xenin-GluPal four hours previously (all at 25 nmol/kgbody weight). Plasma glucose AUC values are also included. Values represent mean ± SEM for 8 mice. ^{*}P<0.05 compared with saline alone.

### EXPERIMENTAL EXAMPLE

### Purification and characterisation of native xenin and xenin-GluPal

Native xenin (MLTKFETKSARVKGLSFHPKRPWIL - SEQ ID NO. 1, also referred to herein as xenin-25) and xenin-GluPal (native xenin with a GluPal modification ((γ-L-Glutamoyl(N-α-hexadecanoyl))) on lysine (K) 13 (the epsilon nitrogen of the lysine residue is substituted with the GluPal modification)) were synthesised by conventional Fmoc peptide chemistry protocols (GL Biochem Ltd., Shanghai, China) and HPLC purification showed retention times of approximately 16.9 and 25.9 minutes, respectively (data provided by company). Xenin-GluPal and Xenin-25[Lys¹³PAL] are synonyms. Following MALDI-ToF MS analysis (samples were mixed with α-cyano-4-hydroxycinnamic acid and applied to a Voyager-DE BioSpectrometry Workstation and mass-to-charge (m/z) ratio verses peak intensity recorded), native xenin exhibited an experimental molecular mass of 2970.3 Da compared to theoretical mass of 2971.6 Da. Similarly, xenin-GluPal showed a MALDI-ToF MS spectrum of 3337.6 Da which closely matched its expected theoretical value of 3338.0 Da. This MALDI-ToF MS analysis confirmed that both native xenin and xenin-GluPal had been synthesised correctly (data not shown). The difference of 367.3 Da between the experimental molecular mass of native xenin and the experimental molecular mass of xenin-GluPal is the expected molecular weight for the gamma-glutamyl spacer plus fatty acid moiety (i.e. the GluPal modification).

### Actions of DPP-IV on native xenin

Dipeptidyl peptidase-4 (DPP-4) was used to determine the degradation properties and identify any degradation products. Each peptide (30 µg) was incubated at 37°C DPP-4 with 455 µl 50 mM triethanolamine-HCl (TEA) pH 7.8 buffer, and the reaction was stopped with 5 µl 10% (v/v) TFA. Time 0, 15, 30, 60, 120, 240 and 480 minutes degradation profiles were measured and HPLC was carried out to identify the degradation pattern and degradation products.

When native xenin was incubated in the presence of DPP-IV for 0, 15, 30, 60, 120, 240 and 480 minutes, HPLC analysis revealed that no degradation products (or peak fragments) were produced, thus indicating that native xenin was completely resistant to degradation by DPP-IV over the time period observed (data not shown). Furthermore, all peptide peaks exhibited similar retention times and MALDI-ToF analysis demonstrated that experimental masses observed for each peptide peak was similar to that of native xenin (2970.3 Da; data not shown). Importantly, to illustrate that the DPP-IV enzyme was functioning properly, the incretin hormone GIP was incubated with DPP-IV for 0 and 120 minutes and HPLC analysis revealed the presence of degradation products (data not shown).

### Actions of native xenin and xenin-GluPal when incubated in the presence of murine plasma

### Protocols

Native xenin and xenin-GluPal (30mL) were incubated (37°C) in the presence of murine plasma (10mL) in 50mM TEA-HCl buffer (pH 7.8) for various time points up to and including 24 hours.

MALDI-ToF Mass Spectrometry: Following solid phase extraction, samples for each degradation time-point were subsequently mixed with α-cyano-4-hydroxycinnamic acid and applied to a Voyager-DE BioSpectrometry Workstation and mass-to-charge (m/z) ratio verses peak intensity recorded.

ESI Mass Spectrometry: Samples for each degradation time-point were applied to the ESI lon-trap MS/MS utilizing a mass-to-charge range (m/z) of 0-2000. Resulting mass spectra were analyzed with the turbo sequest algorithm and degradation fragments sequenced against a fasta database containing the native xenin sequence.

### Results

Over the 24 hour incubation time native xenin was progressively degraded by murine plasma. Indeed, MALDI-ToF MS analysis revealed a number of cleavage products and further analysis by ESI-MS identified these cleavage products as xenin(9-25), xenin(11-25), xenin(14-25) and xenin(18-25). Table 1 summarises the sequences of cleavage products obtained together with their experimental and theoretical masses. Importantly, incubation of native xenin with TEA-HCl buffer alone did not result in any degradation of the peptide. GIP was included as a positive control (data not shown).

**Table 1**

| Amino acid sequence as confirmed by ESI MS/MS sequencing | Cleavage products | Average experimental Molecular weight (Da) | Expected molecular weight (Da) |
|---|---|---|---|
| | Xenin 1-25 | 2969.72 | 2971 |
| | Xenin 9-25 | 1991.27 | 1991.51 |
| R-V-K-G-L-S-F-H-P-K-R-P-W-I-L-OH | Xenin 11-25 | 1833.3 | 1833.35 |
| G-L-S-F-H-P-K-R-P-W-I-L-OH | Xenin 14-25 | 1450.35 | 1449.88 |
| H-P-K-R-P-W-I-L-OH | Xenin 18-25 | 1045.78 | 1045.35 |

The data in Table 2 clearly shows that when xenin-GluPal was incubated with murine plasma, no degradation peaks/fragments were observed over 24 hours. Furthermore, MALD-ToF MS analysis of each of the peptide peaks that were collected indicated that the experimental mass of each peak was similar to that of xenin-GluPal throughout the 24-hr incubation period. These data indicate that, unlike native xenin, xenin-GluPal is stable in the presence of murine plasma.

**Table 2**

| Time-point (hr) | Experimental mass (Da) |
|---|---|
| 0 | 3334.37 |
| 2 | 3335.23 |
| 8 | 3336.38 |
| 12 | 3336.40 |
| 24 | 3337.10 |

The degradation studies have found that in murine plasma, native xenin can be cleaved at the C-terminal side of residues 8, 10, 13 and 17. Thus, the residues immediately flanking these cleavage points, particularly residues 8, 11, 13 and 17, represent useful residues to mutate/modify in order to increase the stability of xenin. Based on their chemical similarity to some of the above mentioned residues, residues 4, 20 and 21 were also selected for mutation/modification.

### Stability of xenin-Gln (SEQ ID NO:16)

The stability of xenin-Gln in murine plasma was also tested. Xenin-Gln is stable in the presence of murine plasma.

### Actions of native xenin,xenin-GluPal and Xenin-Gln on in vitro insulin secretion

BRIN-BD11 cells (ECACC accession number 100330033) were cultured in RPMI 1640 tissue culture medium containing 10% (v/v) foetal calf serum, 1% (v/v) antibiotics (100 U/ml of penicillin and 0.1 mg/ ml of streptomycin). BRIN-BD11 cells were originally derived by means of electrofusion of a New England Deaconess Hospital (NEDH) rat pancreatic beta-cell line with an RINm5F cell line in order to produce an immortal, glucose sensitive, insulin secreting cell line (McClenaghan et.al., Diabetes. 1996 Aug;45(8):1132-40). All cells were maintained in sterile tissue culture flasks (Corning Glass Works, Corning, NY, USA) at 37°C in an atmosphere of 5% CO₂ and 95% air, using an LEEC incubator (Laboratory Technical Engineering, Nottingham, UK).

BRIN-BD11 cells were seeded into 24-well plates at a density of 1x10⁵ cells and allowed to attach to the wells overnight in an incubator. Before acute studies of insulin were carried out, the cells underwent a 40 min preincubation period at 37°C with 1.0ml of Krebs Ringer bicarbonate buffer (115 mM NaCl, 4.7 mM KCI, 1.28 mM CaCl₂.2H₂O, 1.2mM KH₂PO₄, 1.2 mM MgSO₄.7H₂O, 10 mM NaHCO₃, 5 g/l bovine serum albumin (BSA), pH 7.4) supplemented with 1.1 mM glucose. Acute test incubations were performed at 37°C in the presence of 5.6 mM or 16.7mM glucose with concentration range of 10⁻¹² to 10⁻⁶ M and each analogue alone as well as in combination with (D-Ala²)GIP. The incubations were performed for 20 min at 37°C, after which the buffer solution was removed and 2x 200 µl aliquots stored at -20°C until measurement of insulin levels by radioimmunoassay (RIA).

Native xenin and xenin-GluPal stimulated insulin secretion in a concentration-dependent manner in BRIN-BD11 cells at 5.6 mM glucose (Figure 1a). Furthermore, native xenin significantly enhanced insulin production at concentrations of 10⁻⁸ and 10⁻⁶ M (P<0.05 and P<0.001, respectively) compared to glucose alone. Importantly, xenin-GluPal was more potent at stimulating insulin secretion than the native xenin peptide at 10⁻⁸ M and 10⁻⁶ M (P<0.001 and P<0.05, respectively). Figure 1b illustrates the effects of incubating native xenin and xenin-GluPal in the presence of (D-Ala²)GIP in BRIN-BD11 cells at 5.6 mM glucose. Native xenin, xenin-GluPal and (D-Ala²)GIP significantly stimulated insulin secretion in a concentration-dependent manner (P<0.05 to P<0.001) compared to glucose alone. Furthermore, xenin-GluPal was significantly more potent at stimulating insulin secretion at 10⁻¹⁰ and 10⁻⁸ M concentrations compared to native xenin, (D-Ala²)GIP, or a combination of native xenin plus (D-Ala²)GIP. Interestingly, a similar pattern was observed when native xenin and xenin-GluPal were incubated in the presence of exendin-4 (Figure 1c) with the combination of xenin-GluPal and exendin-4 being more potent than exendin-4 alone (P<0.05) or the combination of xenin and exendin-4.

Xenin-25[Lys¹³PAL] significantly (*p* < 0.05 to *p* < 0.001) enhanced the insulinotropic response of (D-Ala²)GIP at both 5.6 and 16.7 mM glucose (Fig 1b and 1d). In addition, the insulinotropic effect of xenin-25[Lys¹³PAL] in combination with (D-Ala²)GIP was significantly (*p* < 0.05 to *p* < 0.01) superior compared to xenin-25 in combination with (D-Ala²)GIP at concentrations of 10⁻¹⁰ and 10⁻⁸ M at both glucose concentrations examined (Fig 1b and 1d). (Notes: xenin-25[Lys¹³PAL] and xenin-GluPal are synonyms. The amino acid sequence of (D-Ala²)GIP is YAEGTFISDYSIAMDKIHQQDFVNWLLAQKGKKNDWKHNITQ, wherein the A (alanine) residue at position 2 is a D-amino acid (SEQ ID NO:17).)

These data demostrate that xenin-GluPal is significantly more potent than native xenin in stimulating insulin secretion in BRIN-BD11 cells both on its own and in combination with other incretins.

In a further experiment, the action of Xenin-Gln (SEQ ID NO:16) on *in vitro* insulin secretion was also assessed. In this further experiment, the materials and methods employed were as described above. Xenin-Gln stimulated insulin secretion in a concentration-dependent manner in BRIN-BD11 cells at 5.6 mM glucose (Figure 1e). Xenin-Gln significantly enhanced insulin production at concentrations of 10⁻⁸ and 10⁻⁶ M (P<0.05 and P<0.001, respectively) compared to glucose alone (Figure 1e). Figure 1e also demonstrates that xenin-Gln stimulates insulin secretion to a greater extent than native xenin in BRIN-BD11 cells.

### Actions of native xenin,xenin-GluPal and xenin-Gln on glucose homeostasis and insulin secretion in high fat fed mice

NIH Swiss male mice (6 weeks old) were housed in an air conditioned room 22±2ºC with a 12 h light: 12 h dark cycle. Animals were maintained on a high fat diet 16 weeks prior to commencement and throughout the study. Drinking water and high fat diet was freely available. All animal studies were performed in accordance with the UK Animals (Scientific Procedures) Act 1986 under project licences held by the inventor.

Freely fed animals were divided into weight-matched groups (n=8) and administered, by intraperitoneal injection, glucose alone (18 mmol/kg body weight) or glucose in combination with each peptide (25 nmol/kg body weight). Blood samples were collected immediately prior to (t=0) and 15, 30 and 60 min post-injection. Whole blood was used to measure glucose values using an Acensia CONTOUR Microfill Blood Glucose Meter and Acensia MICROFILL test strips (Bayer Healthcare, UK). Blood samples for measurement of insulin was collected into fluorine/heparin microcentrifuge tubes (Sarstedt, Numbercht, Germany) at times indicated above and immediately centrifuged using a Beckman microcentrifuge (Beckman Instruments, UK) for 5 min at 13,000 g. Plasma was then aliquoted and stored at -20ºC prior to insulin determination by RIA.

Figure 2a depicts the plasma glucose responses to intraperitoneal (i.p.) administration of glucose alone (18mmol/kg) or in combination with native xenin or xenin-GluPal (each at 25 nmol/kg body weight). Administration of glucose alone resulted in a rapid and protracted rise in plasma glucose concentrations. The plasma glucose excursion following administration of native xenin or xenin-GluPal was not significantly different to animals receiving glucose alone. While there was a trend towards an improved glycaemic profile when plasma glucose AUC (Area Under the Curve) data were analysed, this failed to reach overall significance for either native xenin peptide or the xenin-GluPal peptide. Similarly, the plasma insulin response following administration of native xenin or xenin-GluPal was not significantly different to animals receiving glucose alone in terms of post-injection values (Figure 2b). This was further corroborated by plasma insulin AUC values. These data demonstrate that both native xenin and xenin-GluPal retain their glucose-lowering and insulin-releasing properties when administered acutely in high fat fed mice.

Administration of 25 nmol/kg xenin-25[Lys¹³PAL] to normal (i.e. non-high fat fed) mice resulted in significantly (p < 0.05) reduced plasma glucose levels 60 min post injection compared to glucose control, corroborated by a significantly (p < 0.05) lowered overall glycaemic excursion (Fig 2c). In addition, 0-60 min AUC glucose-stimulated insulin concentrations were significantly elevated in xenin-25[Lys¹³PAL] treated mice compared to both xenin-25 treated (p < 0.05) and control (p < 0.01) mice (Fig 2d).

In further experiments, the action of xenin-Gln (SEQ ID NO:16) on glucose homeostasis and insulin secretion in high fat fed mice was also assessed. In these further experiments, the materials and methods employed were as described above. Administration of 25 nmol/kg xenin-Gln resulted in reduced plasma glucose levels 30 min post injection compared to the glucose alone control (Figure 2e). In addition, 0-60 min AUC plasma glucose values were significantly reduced (P<0.01) compared to glucose control. Importantly, xenin-Gln lowered the overall glycaemic excursion to a greater extent than either native xenin (P<0.05) and xenin-GluPal (P<0.05) (Figure 2e). In addition, plasma insulin concentrations were elevated in xenin-Gln treated mice to a greater extent than both native xenin and glucose alone treated mice (Figure 2f).

### Persistent effects of native xenin and xenin-GluPal on glucose-lowering in high-fat fed mice

NIH Swiss male mice (6 weeks old) were housed in an air conditioned room 22±2ºC with a 12 h light: 12 h dark cycle. Animals were maintained on a high fat diet 16 weeks prior to commencement and throughout the study. Drinking water and high fat diet was freely available. All animal studies were performed in accordance with the UK Animals (Scientific Procedures) Act 1986 under project licences held by the inventor.

Freely fed animals were divided into weight-matched groups (n=8) and administered, by intraperitoneal injection, saline vehicle (0.9% NaCl) or saline in combination with each peptide (25 nmol/kg body weight). Food was then immediately withdrawn and 4 hours later all mice received an intraperitoneal injection of glucose alone (18 mmol/kg body weight). Blood samples were collected immediately prior to (t=0) and 15, 30 and 60 min post glucose injection. Whole blood was used to measure glucose values using an Acensia CONTOUR Microfill Blood Glucose Meter and Acensia MICROFILL test strips (Bayer Healthcare, UK).

As illustrated in Figure 3, when xenin-GluPal was administered 4 hours before an intraperitoneal glucose load was administered, the plasma glucose concentration was significantly lower at 60 minutes post-injection of glucose (P<0.05) than the plasma glucose level in the control mice that had been treated with the saline vehicle only 4 hours before the glucose administration. Furthermore, as evident from the AUC values for plasma glucose, xenin-GluPal was significantly more potent at lowering plasma glucose (P<0.05) than saline alone. Interestingly, native xenin did not elicit any significant effects on plasma glucose when administered 4 hours previously.

Xenin GluPal can be chronically tested in normal, diet-induced and *ob*/*ob* mouse models to examine effects on the following: food intake, bodyweight, plasma glucose, plasma insulin, glucose tolerance and insulinotropic responses, insulin sensitivity, meal test responses, locomotor activity, feeding behaviour patterns, energy expenditure (CLAMS) and DEXA analysis. Terminal assessments of pancreas histology, gene expression, protein content etc may also be performed.

## Claims

1. A peptide
(i) of SEQ ID No. 1, or
(ii) which is an analogue of SEQ ID No.1 which is able to stimulate insulin secretion and which has no more than 7 amino acid substitutions or deletions as compared to the native sequence (SEQ ID No.1),
the peptide having one or more lysine residues substituted with a lipophilic substituent of 8-40 carbon atoms, optionally via a spacer.

2. The peptide of claim 1, wherein said peptide has no amino acid substitutions or deletions as compared to the native sequence.

3. The peptide of claim 1 or claim 2, wherein only one lysine residue is substituted with said lipophilic substituent and/or wherein Lys₁₃ is substituted with said lipophilic substituent.

4. The peptide of any one of claims 1 to 3, wherein said lipophilic substituent is derived from a fatty acid which may be saturated or unsaturated, preferably said lipophilic substituent is derived from palmitic acid.

5. The peptide of any one of claims 1 to 4, wherein said lipophilic substituent is attached to the lysine residue by a spacer, preferably said spacer is Glu.

6. The peptide of any one of claims 1 to 5, wherein said peptide is SEQ ID No.1 having a (γ-L-Glutamoyl(N-a-hexadecanoyl)) modification on lysine (K) 13.

7. A peptide
(i) of SEQ ID No. 1; or
(ii) which is an analogue of SEQ ID No.1 which is able to stimulate insulin secretion and which has no more than 7 amino acid substitutions or deletions as compared to the native sequence (SEQ ID No.1);
in which one or more of Lys₄, Lys₈, Arg₁₁, Lys₁₃, Phe₁₇, Lys₂₀, or Arg₂₁ have been replaced with another amino acid which increases resistance of the peptide to enzymatic degradation.

8. The peptide of claim 7, wherein the replacement amino acid is glutamine or isoleucine, preferably glutamine.

9. The peptide of claim 7 or claim 8, wherein one or more of Lys₄, Lys₈, Arg₁₁, Lys₁₃, Lys₂₀, or Arg₂₁ have been replaced.

10. The peptide of any one of claims 7 to 9, wherein each of Lys₄, Lys₈, Arg₁₁, Lys₁₃, Lys₂₀, and Arg₂₁ have been replaced, preferably said peptide is SEQ ID No.16.

11. A peptide of claim 7 or claim 8, wherein said peptide is selected from the group consisting of SEQ ID No. 2, 3, 4, 5, 6, 7, 8, 9, 10, 11, 12, 13, 14 and 15.

12. A peptide of any one of claims 7 to 9 or claim 11, wherein said peptide comprises one or more substituted lysine residues as defined in any one of claim 1 or claims 3 to 6.

13. A molecule comprising a peptide as defined in any one of claims 1 to 12, or a formulation comprising one or more peptides as claimed in any one of claims 1 to 12 or said molecules in admixture with a suitable diluent, carrier or excipient.

14. A peptide as claimed in any one of claims 1 to 12 or a molecule as claimed in claim 13 or a formulation as claimed in claim 13 for use in therapy, optionally the treatment involves co-administration with another antidiabetic agent, such as insulin, an incretin, or a mimic or analogue thereof, or with another agent for the treatment of Metabolic Syndrome or insulin resistance, or with another antiobesity agent.

15. A peptide as claimed in any one of claims 1 to 12 or a molecule as claimed in claim 13 or a formulation as claimed in claim 13 for use in treating Type 2 diabetes mellitus, insulin dependent diabetes mellitus, insulin resistance, Metabolic Syndrome or obesity, optionally the treatment involves co-administration with another antidiabetic agent, such as insulin, an incretin, or a mimic or analogue thereof, or with another agent for the treatment of Metabolic Syndrome or insulin resistance, or with another antiobesity agent.

## Patentansprüche

1. Peptid
(i) von SEQ ID Nr. 1 oder
(ii) das ein Analogon von SEQ ID Nr. 1 ist, das in der Lage ist, die Insulinsekretion zu stimulieren und das nicht mehr als 7 Aminosäuresubstitutionen oder -deletionen im Vergleich mit der nativen Sequenz (SEQ ID Nr. 1) aufweist,
wobei das Peptid einen oder mehrere Lysinreste aufweist, die mit einem lipophilen Substituenten von 8 - 40 Kohlenstoffatomen, gegebenenfalls über einen Spacer, substituiert sind.

2. Peptid nach Anspruch 1, wobei das Peptid keine Aminosäuresubstitutionen oder - deletionen im Vergleich mit der nativen Sequenz aufweist.

3. Peptid nach Anspruch 1 oder Anspruch 2, wobei nur ein Lysinrest mit dem lipophilen Substituenten substituiert ist und/oder wobei Lys₁₃ mit dem lipophilen Substituenten substituiert ist.

4. Peptid nach einem der Ansprüche 1 bis 3, wobei der lipophile Substituent von einer Fettsäure abgeleitet ist, die gesättigt oder ungesättigt sein kann, wobei der lipophile Substituent bevorzugt von Palmitinsäure abgeleitet ist.

5. Peptid nach einem der Ansprüche 1 bis 4, wobei der lipophile Substituent an den Lysinrest durch einen Spacer angelagert ist, wobei der Spacer bevorzugt Glu ist.

6. Peptid nach einem der Ansprüche 1 bis 5, wobei das Peptid SEQ ID Nr. 1 ist, das eine (γ-L-Glutamoyl(N-α-hexadecanoyl))-Modifikation an Lysin (K) 13 aufweist.

7. Peptid
(i) von SEQ ID Nr. 1; oder
(ii) das ein Analogon von SEQ ID Nr. 1 ist, das in der Lage ist, die Insulinsekretion zu stimulieren und das nicht mehr als 7 Aminosäuresubstitutionen oder -deletionen im Vergleich mit der nativen Sequenz (SEQ ID Nr. 1) aufweist,
wobei eines oder mehrere von Lys₄, Lys₈, Arg₁₁, Lys₁₃, Phe₁₇, Lys₂₀ oder Arg₂₁ mit einer anderen Aminosäure ersetzt worden sind, die die Widerstandsfähigkeit des Peptids gegen enzymatischen Abbau erhöht.

8. Peptid nach Anspruch 7, wobei die ersetzende Aminosäure Glutamin oder Isoleucin, bevorzugt Glutamin, ist.

9. Peptid nach Anspruch 7 oder Anspruch 8, wobei eines oder mehrere von Lys₄, Lys₈, Arg₁₁, Lys₁₃, Lys₂₀ oder Arg₂₁ ersetzt worden sind.

10. Peptid nach einem der Ansprüche 7 bis 9, wobei jedes von Lys₄, Lys₈, Arg₁₁, Lys₁₃, Lys₂₀ und Arg₂₁ ersetzt worden sind, wobei das Peptid bevorzugt SEQ ID Nr. 16 ist.

11. Peptid nach Anspruch 7 oder Anspruch 8, wobei das Peptid aus der Gruppe ausgewählt wird bestehend aus SEQ ID Nr. 2, 3, 4, 5, 6, 7, 8, 9, 10, 11, 12, 13, 14 und 15.

12. Peptid nach einem der Ansprüche 7 bis 9 oder Anspruch 11, wobei das Peptid einen oder mehrere substituierte Lysinreste, wie in irgendeinem von Anspruch 1 oder den Ansprüchen 3 bis 6 definiert, umfasst.

13. Molekül, das ein Peptid wie in einem der Ansprüche 1 bis 12 definiert oder eine Formulierung, die ein oder mehrere Peptide nach einem der Ansprüche 1 bis 12 umfasst, oder die Moleküle in Mischung mit einem geeigneten Verdünnungsmittel, Träger oder einer geeigneten Trägersubstanz umfasst.

14. Peptid nach einem der Ansprüche 1 bis 12 oder Molekül nach Anspruch 13 oder Formulierung nach Anspruch 13 zur Verwendung bei der Therapie, wobei die Behandlung gegebenenfalls die gleichzeitige Verabreichung mit einem anderen Antidiabetikum wie Insulin, einem Inkretin oder einem Imitator oder Analogon davon, oder mit einem anderen Mittel zur Behandlung von metabolischem Syndrom oder Insulinresistenz oder mit einem anderen Mittel gegen Adipositas involviert.

15. Peptid nach einem der Ansprüche 1 bis 12 oder Molekül nach Anspruch 13 oder Formulierung nach Anspruch 13 zur Verwendung zum Behandeln von Diabetes mellitus Typ 2, insulinabhängigem Diabetes mellitus, Insulinresistenz, metabolischem Syndrom oder Adipositas, wobei die Behandlung gegebenenfalls die gleichzeitige Verabreichung mit einem anderen Antidiabetikum wie Insulin, einem Inkretin oder einem Imitator oder Analogon davon, oder mit einem anderen Mittel zur Behandlung von metabolischem Syndrom oder Insulinresistenz oder mit einem anderen Mittel gegen Adipositas involviert.

## Revendications

1. Peptide
(i) de SEQ ID n° 1, ou
(ii) qui est un analogue de SEQ ID n° 1 qui est susceptible de stimuler une sécrétion d'insuline et qui n'a pas plus de 7 substitutions ou délétions d'acides aminés en comparaison à la séquence native (SEQ ID n° 1),
le peptide ayant un ou plusieurs résidus de lysine substitués par un substituant lipophile de 8 à 40 atomes de carbone, de manière facultative par l'intermédiaire d'un espaceur.

2. Peptide selon la revendication 1, dans lequel ledit peptide n'a aucune substitution ou délétion d'acides aminés en comparaison à la séquence native.

3. Peptide selon la revendication 1 ou la revendication 2, dans lequel seul un résidu de lysine est substitué par ledit substituant lipophile et/ou dans lequel Lys₁₃ est substitué par ledit substituant lipophile.

4. Peptide selon l'une quelconque des revendications 1 à 3, dans lequel ledit substituant lipophile est obtenu à partir d'un acide gras qui peut être saturé ou insaturé, de préférence ledit substituant lipophile est obtenu à partir d'acide palmitique.

5. Peptide selon l'une quelconque des revendications 1 à 4, dans lequel ledit substituant lipophile est attaché au résidu de lysine par un espaceur, de préférence ledit espaceur est Glu.

6. Peptide selon l'une quelconque des revendications 1 à 5, dans lequel ledit peptide est SEQ ID n° 1 ayant une modification (y-L-Glutamoyl(N-α-hexadécanoyle)) sur la lysine (K) 13.

7. Peptide
(i) de SEQ ID n° 1 ; ou
(ii) qui est un analogue de SEQ ID n° 1 qui est susceptible de stimuler la sécrétion d'insuline et qui n'a pas plus de 7 substitutions ou délétions d'acides aminés en comparaison à la séquence native (SEQ ID n° 1) ;
dans lequel un ou plusieurs de Lys₄, Lys₈, Arg₁₁, Lys₁₃, Phe₁₇, Lys₂₀, ou Arg₂₁ ont été remplacés par un autre acide aminé qui augmente la résistance du peptide à une dégradation enzymatique.

8. Peptide selon la revendication 7, dans lequel l'acide aminé de remplacement est de la glutamine ou de l'isoleucine, de préférence de la glutamine.

9. Peptide selon la revendication 7 ou la revendication 8, dans lequel un ou plusieurs de Lys₄, Lys₈, Arg₁₁, Lys₁₃, Lys₂₀, ou Arg₂₁ ont été remplacés.

10. Peptide selon l'une quelconque des revendications 7 à 9, dans lequel chacun de Lys₄, Lys₈, Arg₁₁, Lys₁₃, Lys₂₀ et Arg₂₁ ont été remplacés, de préférence ledit peptide est SEQ ID n° 16.

11. Peptide selon la revendication 7 ou la revendication 8, dans lequel ledit peptide est sélectionné à partir du groupe constitué par SEQ ID n° 2, 3, 4, 5, 6, 7, 8, 9, 10, 11, 12, 13, 14 et 15.

12. Peptide selon l'une quelconque des revendications 7 à 9 ou la revendication 11, dans lequel ledit peptide comprend un ou plusieurs résidus de lysine substitués comme défini(s) dans l'une quelconque de la revendication 1 ou des revendications 3 à 6.

13. Molécule comprenant un peptide comme défini dans l'une quelconque des revendications 1 à 12, ou formulation comprenant un ou plusieurs peptides comme revendiqué(s) dans l'une quelconque des revendications 1 à 12 ou lesdites molécules en mélange avec un diluant, vecteur ou excipient approprié.

14. Peptide comme revendiqué dans l'une quelconque des revendications 1 à 12 ou molécule comme revendiquée dans la revendication 13 ou formulation comme revendiquée dans la revendication 13 à utiliser en thérapie, de manière facultative le traitement implique l'administration conjointe d'un autre agent antidiabétique, comme l'insuline, une incrétine, ou un mimétique ou un analogue de celles-ci, ou d'un autre agent pour le traitement du Syndrome Métabolique ou de la résistance à l'insuline, ou d'un autre agent anti-obésité.

15. Peptide comme revendiqué dans l'une quelconque des revendications 1 à 12 ou molécule comme revendiquée dans la revendication 13 ou formulation comme revendiquée dans la revendication 13 à utiliser dans le traitement du diabète sucré de Type 2, du diabète sucré dépendant de l'insuline, de la résistance à l'insuline, du Syndrome Métabolique ou de l'obésité, de manière facultative le traitement implique l'administration conjointe d'un autre agent antidiabétique, comme l'insuline, une incrétine, ou un mimétique ou un analogue de celles-ci, ou d'un autre agent pour le traitement du Syndrome Métabolique ou de la résistance à l'insuline, ou d'un autre agent anti-obésité.


### SEQUENCE LISTING

<110> University of Ulster
<120> Peptides and peptide derivatives based on xenin
<130> 59.15.110798/03
<150> GB1200509.6
   <151> 2012-01-12
<150> GB1207895.2
   <151> 2012-05-04
<160> 17
<170> PatentIn version 3.5
<210> 1
   <211> 25
   <212> PRT
   <213> human
<400> 1
   Met Leu Thr Lys Phe Glu Thr Lys Ser Ala Arg Val Lys Gly Leu Ser
   1               5                   10                  15
   Phe His Pro Lys Arg Pro Trp Ile Leu
               20                  25
<210> 2
   <211> 25
   <212> PRT
   <213> Artificial Sequence
<220>
   <223> xenin point mutant
<400> 2
   Met Leu Thr Lys Phe Glu Thr Gln Ser Ala Arg Val Lys Gly Leu Ser
   1               5                   10                  15
   Phe His Pro Lys Arg Pro Trp Ile Leu
               20                  25
<210> 3
   <211> 25
   <212> PRT
   <213> Artificial Sequence
<220>
   <223> xenin point mutant
<400> 3
   Met Leu Thr Lys Phe Glu Thr Ile Ser Ala Arg Val Lys Gly Leu Ser
   1               5                   10                  15
   Phe His Pro Lys Arg Pro Trp Ile Leu
               20                  25
<210> 4
   <211> 25
   <212> PRT
   <213> Artificial Sequence
<220>
   <223> xenin point mutant
<400> 4
   Met Leu Thr Lys Phe Glu Thr Lys Ser Ala Gln Val Lys Gly Leu Ser
   1               5                   10                  15
   Phe His Pro Lys Arg Pro Trp Ile Leu
               20                  25
<210> 5
   <211> 25
   <212> PRT
   <213> Artificial Sequence
<220>
   <223> xenin point mutant
<400> 5
   Met Leu Thr Lys Phe Glu Thr Lys Ser Ala Ile Val Lys Gly Leu Ser
   1               5                   10                  15
   Phe His Pro Lys Arg Pro Trp Ile Leu
               20                  25
<210> 6
   <211> 25
   <212> PRT
   <213> Artificial Sequence
<220>
   <223> xenin point mutant
<400> 6
   Met Leu Thr Lys Phe Glu Thr Lys Ser Ala Arg Val Gln Gly Leu Ser
   1               5                   10                  15
   Phe His Pro Lys Arg Pro Trp Ile Leu
               20                  25
<210> 7
   <211> 25
   <212> PRT
   <213> Artificial Sequence
<220>
   <223> xenin point mutant
<400> 7
   Met Leu Thr Lys Phe Glu Thr Lys Ser Ala Arg Val Ile Gly Leu Ser
   1               5                   10                  15
   Phe His Pro Lys Arg Pro Trp Ile Leu
               20                  25
<210> 8
   <211> 25
   <212> PRT
   <213> Artificial Sequence
<220>
   <223> xenin point mutant
<400> 8
   Met Leu Thr Lys Phe Glu Thr Lys Ser Ala Arg Val Lys Gly Leu Ser
   1               5                   10                  15
   Gln His Pro Lys Arg Pro Trp Ile Leu
               20                  25
<210> 9
   <211> 25
   <212> PRT
   <213> Artificial Sequence
<220>
   <223> xenin point mutant
<400> 9
   Met Leu Thr Lys Phe Glu Thr Lys Ser Ala Arg Val Lys Gly Leu Ser
   1               5                   10                  15
   Ile His Pro Lys Arg Pro Trp Ile Leu
               20                  25
<210> 10
   <211> 25
   <212> PRT
   <213> Artificial Sequence
<220>
   <223> xenin point mutant
<400> 10
   Met Leu Thr Gln Phe Glu Thr Lys Ser Ala Arg Val Lys Gly Leu Ser
   1               5                   10                  15
   Phe His Pro Lys Arg Pro Trp Ile Leu
               20                  25
<210> 11
   <211> 25
   <212> PRT
   <213> Artificial Sequence
<220>
   <223> xenin point mutant
<400> 11
   Met Leu Thr Ile Phe Glu Thr Lys Ser Ala Arg Val Lys Gly Leu Ser
   1               5                   10                  15
   Phe His Pro Lys Arg Pro Trp Ile Leu
               20                  25
<210> 12
   <211> 25
   <212> PRT
   <213> Artificial Sequence
<220>
   <223> xenin point mutant
<400> 12
   Met Leu Thr Lys Phe Glu Thr Lys Ser Ala Arg Val Lys Gly Leu Ser
   1               5                   10                  15
   Phe His Pro Gln Arg Pro Trp Ile Leu
               20                  25
<210> 13
   <211> 25
   <212> PRT
   <213> Artificial Sequence
<220>
   <223> xenin point mutant
<400> 13
   Met Leu Thr Lys Phe Glu Thr Lys Ser Ala Arg Val Lys Gly Leu Ser
   1               5                   10                  15
   Phe His Pro Ile Arg Pro Trp Ile Leu
               20                  25
<210> 14
   <211> 25
   <212> PRT
   <213> Artificial Sequence
<220>
   <223> xenin point mutant
<400> 14
   Met Leu Thr Lys Phe Glu Thr Lys Ser Ala Arg Val Lys Gly Leu Ser
   1               5                   10                  15
   Phe His Pro Lys Gln Pro Trp Ile Leu
               20                  25
<210> 15
   <211> 25
   <212> PRT
   <213> Artificial Sequence
<220>
   <223> xenin point mutant
<400> 15
   Met Leu Thr Lys Phe Glu Thr Lys Ser Ala Arg Val Lys Gly Leu Ser
   1               5                   10                  15
   Phe His Pro Lys Ile Pro Trp Ile Leu
               20                  25
<210> 16
   <211> 25
   <212> PRT
   <213> Artificial Sequence
<220>
   <223> xenin point mutant
<400> 16
   Met Leu Thr Gln Phe Glu Thr Gln Ser Ala Gln Val Gln Gly Leu Ser
   1               5                   10                  15
   Phe His Pro Gln Gln Pro Trp Ile Leu
               20                  25
<210> 17
   <211> 42
   <212> PRT
   <213> Artificial Sequence
<220>
   <223> (D-Ala²)GIP
<220>
   <221> SITE
   <222> (2)..(2)
   <223> The A residue at position 2 is a D-amino acid
<400> 17
   Tyr Ala Glu Gly Thr Phe Ile Ser Asp Tyr Ser Ile Ala Met Asp Lys
   1               5                   10                  15
   Ile His Gln Gln Asp Phe Val Asn Trp Leu Leu Ala Gln Lys Gly Lys
               20                  25                  30
   Lys Asn Asp Trp Lys His Asn Ile Thr Gln
           35                  40